(19) Europäisches Patentamt
European Patent Office
Office européen des brevets

(11) Veröffentlichungsnummer: **0 056 113**
**B1**

# (12) EUROPÄISCHE PATENTSCHRIFT

(45) Veröffentlichungstag der Patentschrift: **29.05.85**

(51) Int. Cl.$^4$: **C 07 C 121/46**, C 07 C 121/66, C 09 K 19/00 // C07C120/00, C07C120/04

(21) Anmeldenummer: **81110073.4**

(22) Anmeldetag: **02.12.81**

(54) **Cyclohexylcarbonitrilderivate, diese enthaltende Dielektrika und elektrooptisches Anzeigeelement.**

(30) Priorität: **07.01.81 DE 3100142**

(43) Veröffentlichungstag der Anmeldung: **21.07.82 Patentblatt 82/29**

(45) Bekanntmachung des Hinweises auf die Patenterteilung: **29.05.85 Patentblatt 85/22**

(84) Benannte Vertragsstaaten: **CH DE FR GB LI**

(56) Entgegenhaltungen:
**EP - A - 0 023 730**
**DE - A - 2 701 591**
**DE - A - 2 702 598**

**Mol. Cryst. Liq. Cryst. (Letters) Vol. 56 (1980) pp. 157-161**

(73) Patentinhaber: **Merck Patent Gesellschaft mit beschränkter Haftung, Frankfurter Strasse 250, D-6100 Darmstadt (DE)**

(72) Erfinder: **Praefcke, Klaus, Prof. Dr., Kranzallee 62, D-1000 Berlin 19 (DE)**
Erfinder: **Schmidt, Dietmar, Gardeschützenweg 124, D-1000 Berlin (DE)**
Erfinder: **Eidenschink, Rudolf, Dr., Erlenweg 17, D-6110 Dieburg (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

**Beschreibung**

Für elektrooptische Anzeigeelemente werden in großem Umfang die Eigenschaften nematischer oder nematisch-cholesterischer flüssigkristalliner Materialien ausgenutzt, ihre optischen Eigenschaften wie Lichtabsorption, Lichtstreuung, Doppelbrechung, Reflexionsvermögen oder Farbe unter dem Einfluß elektrischer Felder signifikant zu verändern. Die Funktion derartiger Anzeigeelemente beruht dabei beispielsweise auf den Phänomenen der dynamischen Streuung, der Deformation aufgerichteter Phasen, dem Schadt-Helfrich-Effekt in der verdrillten Zelle oder dem cholesterisch-nematischen Phasenübergang.

Für die technische Anwendung dieser Effekte in elektronischen Bauelementen werden flüssigkristalline Dielektrika benötigt, die einer Vielzahl von Anforderungen genügen müssen. Besonders wichtig sind hier die chemische Beständigkeit gegenüber Feuchtigkeit, Luft und physikalischen Einflüssen wie Wärme, Strahlung im infraroten, sichtbaren und ultravioletten Bereich und elektrische Gleich- und Wechselfelder. Ferner wird von technisch verwendbaren flüssigkristallinen Dielektrika eine flüssigkristalline Mesophase im Temperaturbereich von mindestens +10°C bis +50°C, bevorzugt von 0°C bis 60°C, und eine möglichst niedrige Viskosität bei Raumtemperatur, die vorzugsweise nicht mehr als $70 \cdot 10^{-3}$ Pa · s betragen soll, gefordert. Schließlich dürfen sie im Bereich des sichtbaren Lichtes keine Eigenabsorption aufweisen, d. h. sie müssen farblos sein.

Es ist bereits eine Anzahl von flüssigkristallinen Verbindungen bekannt, die den an Dielektrika für elektronische Bauelemente gestellten Stabilitätsanforderungen genügen und auch farblos sind. Hierzu gehören insbesondere die in der DE-OS 2 139 628 beschriebenen p,p'-disubstituierten Benzoesäurephenylester und die in der DE-OS 2 636 684 beschriebenen p,p'-disubstituierten Phenylcyclohexanderivate. In beiden genannten Verbindungsklassen wie auch in anderen bekannten Reihen von Verbindungen mit flüssigkristalliner Mesophase gibt es keine Einzelverbindungen, die in dem geforderten Temperaturbereich von 10°C bis 60°C eine flüssigkristalline nematische Mesophase ausbilden. Es werden daher in der Regel Mischungen von zwei oder mehreren Verbindungen hergestellt, um als flüssigkristalline Dielektrika verwendbare Substanzen zu erhalten. Hierzu mischt man gewöhnlich mindestens eine Verbindung mit niedrigem Schmelz- und Klärpunkt mit einer anderen mit deutlich höherem Schmelz- und Klärpunkt. Hierbei wird normalerweise ein Gemisch erhalten, dessen Schmelzpunkt unter dem der niedriger schmelzenden Komponente liegt, während der Klärpunkt zwischen den Klärpunkten der Komponenten liegt. Trotzdem bereitet die Herstellung optimaler Dielektrika immer wieder Schwierigkeiten, da die Komponenten mit den hohen Schmelz- und Klärpunkten den Gemischen häufig auch eine hohe Viskosität verleihen. Dadurch werden die Schaltzeiten der damit hergestellten elektrooptischen Anzeigeelemente in unerwünschter Weise verlängert. Ferner treten oft Probleme dadurch auf, daß die Löslichkeit der verschiedenen Komponenten ineinander, insbesondere bei Raumtemperatur oder tieferen Temperaturen nur sehr begrenzt ist.

Ein Cyclohexylcarbonitrilderivat der Formel A

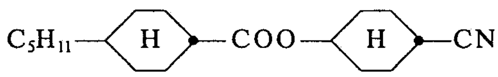

(A)

ist bereits bekannt (M.A. Osman, L. Revesz, Mol. Cryst. Liq. Cryst., Vol. 56 (Letters), (1980), pp. 157–161). Der Klärpunkt dieser monotropen Verbindung liegt jedoch nahezu 44° unterhalb des Schmelzpunktes, während die entsprechende Dialkylverbindung trans-4-n-Pentylcyclohexancarbonsäure-(4-n-propylcyclohexylester) eine nematische Phase im Temperaturbereich von 36,8 bis 52,1° hat.

Der Erfindung liegt die Aufgabe zugrunde, flüssigkristalline Dielektrika herzustellen, die eine nematische Phase im geforderten Temperaturbereich aufweisen und in Flüssigkristallzellen bei Raumtemperatur ausreichend kurze Schaltzeiten ermöglichen.

Es wurde nun gefunden, daß die Cyclohexylcarbonitrilderivate der Formel (I)

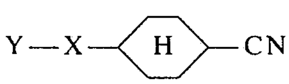

(I)

worin

Y 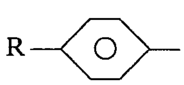 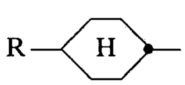 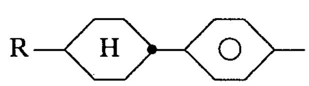

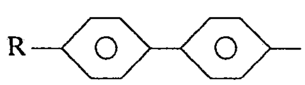 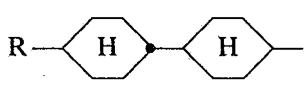

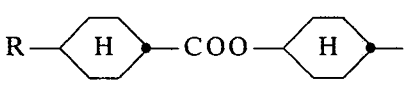 oder 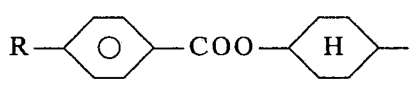

X $-COO-$, $-O-CO-$, $-CH_2O-$, $-OCH_2-$ oder $-CH_2CH_2-$ und
R Alkyl oder Alkoxy mit 1–12 Kohlenstoffatomen

bedeutet, wobei jedoch Y nicht

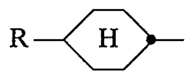 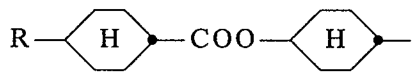

oder

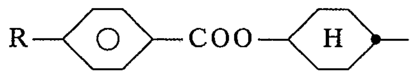

bedeutet, wenn X $-COO-$ oder $-O-CO-$ ist,
vorzüglich als Komponenten flüssigkristalliner Dielektrika geeignet sind. Dabei besitzen diese Verbindungen einen außerordentlichen breiten Anwendungsbereich; in Abhängigkeit von der Auswahl der Substituenten können die Verbindungen der Formel (I) sowohl als Basismaterialien dienen, aus denen flüssigkristalline Dielektrika ausschließlich oder zum überwiegenden Teil zusammengesetzt sind, es können aber auch Verbindungen der Formel (I) in geringeren Anteilen von beispielsweise 2 bis 45 Gewichtsprozent flüssigkristallinen Basismaterialien aus anderen Verbindungsklassen zugesetzt werden, um so Dielektrika mit einer verbreiterten flüssigkristallinen Mesophase herzustellen oder die Größe der dielektrischen Anisotropie eines solchen Dielektrikums zu beeinflussen.

Durch geeignete Auswahl der Gruppen X und Y lassen sich mit den Verbindungen der Formel (I) sowohl Dielektrika mit ausgeprägter positiver dielektrischer Anisotropie zur Verwendung in Anzeigeelementen auf der Basis der verdrillten nematischen Zelle oder des cholesterisch-nematischen Phasenübergangs herstellen, es können aber auch Dielektrika mit von Null nur wenig verschiedener dielektrischer Anisotropie hergestellt werden, die in Anzeigeelementen auf der Basis der dynamischen Streuung oder der Deformation aufgerichteter Phasen (DAP-Effekt) verwendet werden.

Die Verbindungen der Formel (I) sind in reinem Zustand farblos und bilden nematische Mesophasen in einem erstaunlich breiten und für die elektrooptische Verwendung günstig gelegenen Temperaturbereich.

Gegenstand der Erfindung sind somit die Cyclohexylcarbonitrilderivate der Formel (I) und ihre Verwendung als Komponenten flüssigkristalliner Dielektrika. Gegenstand der Erfindung sind weiterhin flüssigkristalline Dielektrika mit einem Gehalt an mindestens einem Cyclohexylcarbonitrilderivat der Formel (I) sowie elektrooptische Anzeigeelemente auf der Basis einer Flüssigkristallzelle, die ein derartiges flüssigkristallines Dielektrikum enthalten.

Die erfindungsgemäßen Cyclohexylcarbonitrilderivate umfassen der 4-Alkyl- und Alkoxybenzoesäure-(trans-4-cyanocyclohexyl)-ester der Formel (Ia),

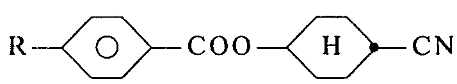 (Ia)

die 4-(trans-4-Alkyl- und Alkoxycyclohexyl)-benzoesäure-(trans-4-cyanocyclohexyl)-ester der Formel (Ib),

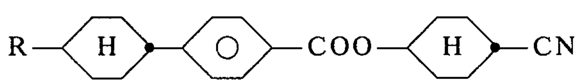 (Ib)

die 4-Alkyl-Alkoxybiphenyl-4-carbonsäure-(trans-4-cyanocyclohexyl)-ester der Formel (Ic),

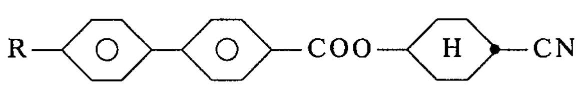 (Ic)

die all-trans-4-(4-Alkyl- und Alkoxycyclohexyl)-cyclohexancarbonsäure-(4-cyanocyclohexyl)-ester der Formel (Id),

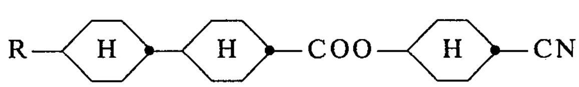 (Id)

die trans-4-Cyanocyclohexancarbonsäure-(4-alkyl- und alkoxyphenyl)-ester der Formel (If),

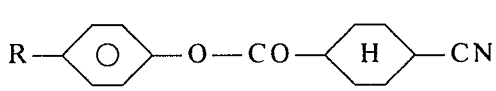 (If)

die trans-4-Cyanocyclohexancarbonsäure-[-(trans-4-alkyl- und alkoxycyclohexyl)-phenyl]-ester der Formel (Ih),

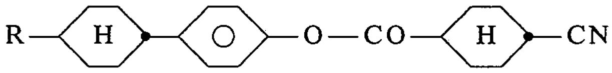

(Ih)

die all-trans-4-Cyanocyclohexancarbonsäure-[4-(4-alkyl- und alkoxycyclohexyl)-cyclohexyl]-ester der Formel (Ii),

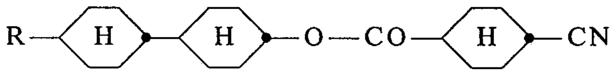

(Ii)

die trans-4-Cyanocyclohexancarbonsäure-(4-alkyl- und alkoxybiphenyl-4-yl)-ester der Formel (Il),

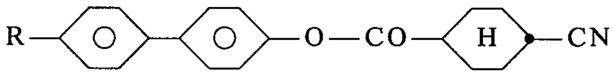

(Il)

die 4-Alkyl- und Alkoxybenzyl-(trans-4-cyanocyclohexyl)-ether der Formel (Im),

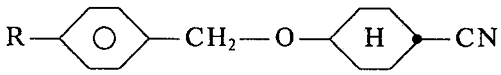

(Im)

die trans-4-Alkyl- und Alkoxycyclohexylmethyl-(trans-4-cyanocyclohexyl)-ether der Formel (In),

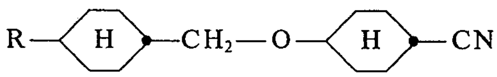

(In)

die 4-(trans-4-Alkyl- und Alkoxycyclohexyl)-benzyl-(trans-4-cyanocyclohexyl)-ether der Formel (Io),

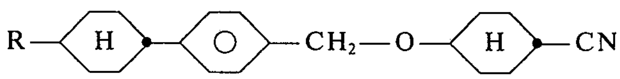

(Io)

die 4-(4-Alkyl- und Alkoxyphenyl)-benzyl-(trans-4-cyanocyclohexyl)-ether der Formel (Ip),

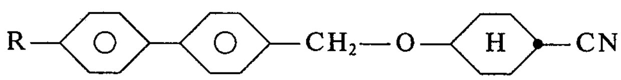

(Ip)

die all-trans-4-(4-Alkyl- und Alkoxycyclohexyl)-cyclohexylmethyl-(4-cyanocyclohexyl)-ether der Formel (Iq),

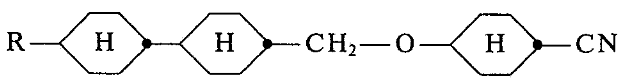

(Iq)

die all-trans-4-(4-Alkyl- und Alkoxycyclohexanoyloxy)-cyclohexylmethyl-(4-cyanocyclohexyl)-ether der Formel (Ir),

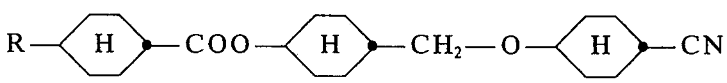

(Ir)

die trans-4-Cyanocyclohexylmethyl-(4-alkyl- und alkoxyphenyl)-ether der Formel (Is),

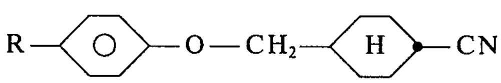

(Is)

die trans-4-Cyanocyclohexylmethyl-(trans-4-alkyl- und alkoxycyclohexyl)-ether der Formel (It),

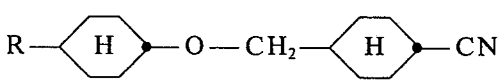

(It)

die trans-4-Cyanocyclohexylmethyl-[4-(trans-4-alkyl- und alkoxycyclohexyl)-phenyl]-ether der Formel (Iu),

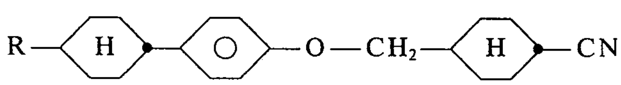

(Iu)

die all-trans-4-Cyanocyclohexylmethyl-[4-(4-alkyl- und alkoxycyclohexyl)-cyclohexyl]-ether der Formel (Iv),

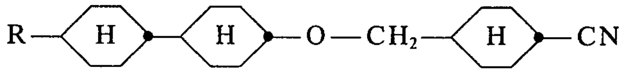

(Iv)

die trans-4-Cyanocyclohexylmethyl-(4-alkyl- und alkoxybiphenyl-4-yl)-ether der Formel (Iw),

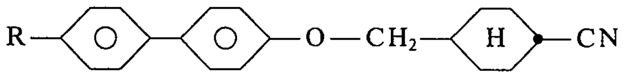

(Iw)

die all-trans-4-Cyanocyclohexylmethyl-[4-(4-alkyl- und alkoxycyclohexanoyloxy)-cyclohexyl]-ether der Formel (Ix),

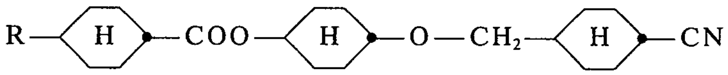

(Ix)

die 1-(4-Alkyl- und Alkoxyphenyl)-2(trans-4-cyanocyclohexyl)-ethane der Formel (Iy),

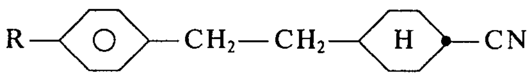

(Iy)

die 1-(trans-4-Alkyl- und alkoxycyclohexyl)-2-(trans-4-cyanocyclohexyl)-ethane der Formel (Iz),

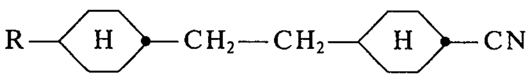

(Iz)

die 1-[4-(trans-4-Alkyl- und alkoxycyclohexyl)-phenyl]-2-(trans-4-cyanocyclohexyl)-ethane der Formel (Iaa),

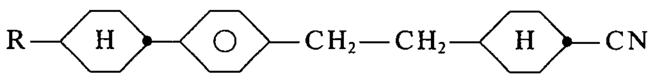

(Iaa)

die all-trans-1-[4-(4-Alkyl- und alkoxycyclohexyl)-cyclohexyl]-2-(4-cyanocyclohexyl)-ethane der Formel (Ibb),

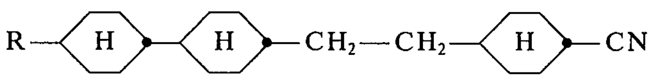

(Ibb)

die 1-(4-Alkyl- und alkoxybiphenyl-4-yl)-2-(trans-4-cyanocyclohexyl)-ethane der Formel (Icc),

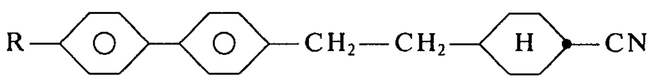

(Icc)

die all-trans-1-[4-(4-Alkyl- und Alkoxycyclohexanoyloxy)-cyclohexyl]-2-(4-cyanocyclohexyl)-ethane der Formel (Idd),

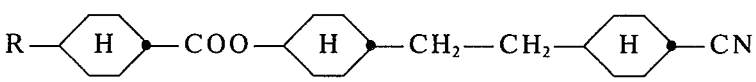

(Idd)

die trans-4-(4-Alkyl- und Alkoxybenzoyloxy)-cyclohexylmethyl-(trans-4-cyanocyclohexyl)-ether der Formel (Igg),

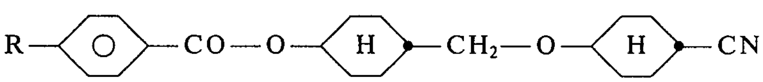

(Igg)

die trans-4-Cyanocyclohexylmethyl-[trans-4-(4-alkyl- und alkoxybenzoyloxy)-cyclohexyl]-ether der Formel (Ihh), und

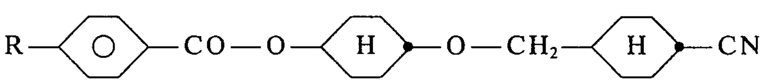

(Ihh)

die 1-[trans-4-(4-Alkyl- und Alkoxybenzoyloxy)-cyclohexyl]-2-(trans-4-cyanocyclohexyl)-ethane der Formel (Iii).

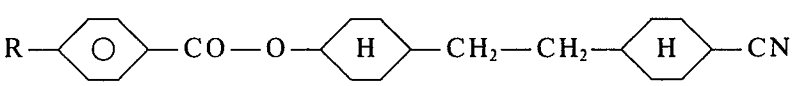

(Iii)

In den Verbindungen der Formel (I) kann der Alkyl- bzw. Alkoxyrest R geradkettig oder verzweigt sein. Wenn er geradkettig ist, also Methyl, Ethyl, n-Propyl, n-Butyl, n-Pentyl, n-Hexyl, n-Heptyl, n-Octyl, n-Nonyl, n-Decyl, n-Undecyl oder n-Dodecyl bedeutet, besitzen die dadurch charakterisierten Verbindungen in der Regel höhere Klärpunkte als die mit verzweigten Flügelgruppe R und sind deswegen bevorzugt. Verbindungen der Formel (I) mit einer verzweigten Flügelgruppe R sind gelegentlich wegen einer besseren Löslichkeit in den üblichen flüssigkristallinen Basismaterialien von Bedeutung, insbesondere aber als chirale Dotierstoffe, wenn sie durch die Kettenverzweigung optische Aktivität besitzen. Solche verzweigten Flügelgruppen R enthalten nicht mehr als eine Kettenverzweigung. Bevorzugte verzweigte Reste R sind die, in denen sich an einer längeren Kohlenstoffkette in 1-, 2- oder 3-Stellung eine Methyl- oder Ethylgruppe befindet, beispielsweise 2-Methylpropyl, 2-Methylbutyl, 3-Methylbutyl, 2-Methylpentyl, 3-Methylpentyl, 2-Ethylhexyl oder 1-Methylhexyl sowie die entsprechenden Alkoxygruppen. Die bevorzugten Kettenlängen der Flügelgruppen R sind je nach der Struktur der Gruppe Y unterschiedlich. Wenn Y 4-R-Phenyl bedeutet, ist R vorzugsweise Alkyl mit 2 bis 11, insbesondere 3 bis 8 Kohlenstoffatomen oder Alkoxy mit 1 bis 10 Kohlenstoffatomen. Wenn Y 4-R-Cyclohexyl ist, sind als Alkylgruppen solche mit 3 bis 10 Kohlenstoffatomen und als Alkoxygruppen solche mit 2 bis 8 Kohlenstoffatomen bevorzugt. Wenn die Gruppe Y 4-R-Biphenyl-4'-yl, 4-(4-R-Cyclohexyl)-phenyl bzw. 4-(4-R-Cyclohexyl)-cyclohexyl ist, ist die Flügelgruppe R vorzugsweise Alkyl mit 2 bis 8 Kohlenstoffatomen oder Alkoxy mit 1 bis 6 Kohlenstoffatomen. Diese Reste R sind ebenfalls die bevorzugten, wenn Y 4-(4-Alkyl- bzw. Alkoxycyclohexanoyloxy)-cyclohexyl bedeutet.

Die erfindungsgemäßen Verbindungen werden in für derartige Substanzen üblicher Weise hergestellt. So werden die Ester der Formeln (Ia) bis (Il) vorzugsweise dargestellt, indem man ein trans-4-Cyanocyclohexanderivat der Formel (II)

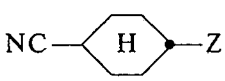

(II)

worin

Z OH, OMe, COOH oder ein reaktionsfähiges Derivat der Carboxylgruppe und
Me ein Äquivalent eines Metallkations bedeutet,

bei einer Temperatur zwischen −50°C und +250°C, gegebenenfalls in Gegenwart eines organischen Lösungsmittels und/oder eines üblichen Veresterungskatalysators, mit einer Verbindung der Formel (III)

Z'–Y (III)

worin

Z' COOH oder ein reaktionsfähiges Derivat der Carboxylgruppe bedeutet, wenn Z OH oder OMe ist, und OH oder OMe bedeutet, wenn Z COOH oder ein reaktionsfähiges Carboxylgruppenderivat ist,

umsetzt.

Dabei werden die Verbindungen der Teilformeln (Ia) bis (Id) erhalten, wenn Z eine Hydroxylgruppe oder eine Alkoholatgruppe, vorzugsweise eine Alkalimetall- oder Erdalkalimetallalkoholatgruppe ist. Die Gruppe Z' in den Verbindungen der Formel (III) ist in diesem Fall —COOH oder ein reaktionsfähiges Carboxylgruppenderivat, vorzugsweise —CO-Halogen, insbesondere —COCl oder —COBr, —COO-Niederalkyl, insbesondere $-COOCH_3$, oder eine Anhydridgruppierung, insbesondere ein gemischtes Anhydrid wie zum Beispiel $-COOCOCH_3$.

Entsprechend entstehen die Verbindungen der Teilformeln (If) bis (Il), wenn Z COOH oder ein reaktionsfähiges Carbonxylgruppenderivat und Z' eine Hydroxyl- oder Alkoholatgruppe ist. Die Reaktionsbedingungen werden weitgehend von der Natur der Gruppen Z und Z' bestimmt: so wird eine Carbonsäure mit einem Alkohol (Z, Z' = COOH, OH) in der Regel in Gegenwart einer starken Säure, beispielsweise einer Mineralsäure wie Salzsäure oder Schwefelsäure, umgesetzt. Eine bevorzugte Reaktionsweise ist die Umsetzung eines Säureanhydrids oder insbesondere eines Säurechlorids mit einem Alkohol (Z, Z' = COCl, OH). Vorzugsweise werden diese Veresterungsreaktionen in einem basischen Milieu durchgeführt, wobei als Basen insbesondere Alkalimetallhydroxide wie Natrium- oder Kaliumhydroxid, Alkalimetallcarbonate bzw. -hydrogencarbonate wie Natriumcarbonat, Natriumhydrogencarbonat, Kaliumcarbonat oder Kaliumhydrogencarbonat, Alkalimetallacetate wie Natrium- oder Kaliumacetat, Erdalkalimetallhydroxide wie Calciumhydroxid oder organische Basen wie Triethylamin, Pyridin, 4-Dimethylaminopyridin oder Chinolin von Bedeutung sind.

Die Veresterungen werden vorteilhaft in Gegenwart eines inerten Lösungsmittels durchgeführt. Gut geeignet sind insbesondere Ether wie Diethylether, Di-n-butylether, Tetrahydrofuran, Dioxan oder Anisol, Ketone wie Aceton, Butanon, Pentanon-(3) oder Cyclohexanon, Amide wie Dimethylformamid oder Hexamethylphosphorsäuretriamid, Kohlenwasserstoffe wie Bezol, Toluol oder Xylol,

Halogenkohlenwasserstoffe wie Tetrachlorkohlenstoff oder Dichlormethan und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuß einer verwendeten organischen Base, zum Beispiel Pyridin, Chinolin oder Triethylamin als Lösungsmittel für die Veresterung angewandt werden. Prinzipiell können die Veresterungsreaktionen auch in Abwesenheit eines Lösungsmittels, zum Beispiel durch einfaches Erhitzen der Komponenten in Gegenwart von Natriumacetat, durchgeführt werden.

Die Reaktionstemperatur liegt gewöhnlich zwischen −50°C und +250°C, vorzugsweise zwischen −20°C und +80°C. Bei diesen Temperaturen sind die Veresterungsreaktionen in der Regel nach 15 Minuten bis 48 Stunden beendet.

Eine weitere bevorzugte Ausführungsform besteht darin, daß man den zu veresternden Alkohol der Formel II bzw. III (Z bzw. Z' = OH) zunächst in sein Natrium- oder Kaliumsalz überführt, zum Beispiel durch Behandeln mit ethanolischer Natron- oder Kalilauge, dieses Salz isoliert und zusammen mit Natriumhydrogenkarbonat oder Kaliumcarbonat unter Rühren in Aceton oder Diethylether suspendiert, und diese Suspension tropfenweise unter Rühren mit einer Lösung eines Säurechlorids oder Anhydrids in Diethylester, Aceton oder Dimethylformamid versetzt. Dabei wird das Reaktionsgemisch auf einer Temperatur zwischen −250°C und +20°C, vorzugsweise bei −10°C bis +20°C gehalten. Bei dieser Arbeitsweise ist die Veresterungsreaktion gewöhnlich nach 15 bis 50 Minuten beendet.

Die Ether der Formeln (Im) bis (Iw) mit Ausnahme der Verbindungen (Ir) werden beispielsweise hergestellt, indem man in einem Ester der Formel (IV)

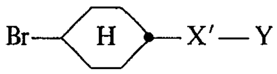

(IV)

worin X' −COO− oder −OCO− bedeutet und Y die bei Formel (I) angegebene Bedeutung − mit Ausnahme der trans-4-(trans-4-R-Cyclohexanoyloxy)-cyclohexylgruppierung und der trans-4-(4-R-Benzoyloxy)-cyclohexylgruppierung − besitzt, die Ester-Carbonylgruppe zur Methylgruppe reduziert, zum Beispiel mit Natriumborhydrid und Bortrifluorid, und anschließend das Bromatom in an sich üblicher Weise, zum Beispiel durch Umsetzung mit einem Metallcyanid in wäßrig-alkoholischer Lösung gegen die Cyanogruppe austauscht.

Bei der Synthese der Ether der Formeln (Ir), (Ix), (Igg) und (hh) wird in analoger Weise zunächst ein Ester der Formel (V)

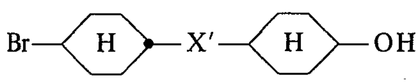

(V)

worin X' die bei Formel (IV) angegebene Bedeutung besitzt, reduziert, und Br gegen CN ausgetauscht; anschließend wird die Hydroxygruppe mit 4-R-Cyclohexancarbonsäure oder einem reaktiven Derivat dieser Säure in an sich üblicher Weise verestert.

Die 1,2-disubstituierten Ethane der Formeln (Iy) bis (Icc) werden beispielsweise hergestellt, indem man 4-Cyanocyclohexanon mit einer Grignard-Verbindung der Formel (VI)

$$Hal{-}Mg{-}CH_2{-}CH_2{-}Y \qquad (VI)$$

worin Hal Cl, Br oder J bedeutet und Y die bei Formel (I) angegebene Bedeutung − mit Ausnahme der 4-(trans-4-R-Cyclohexanoyloxy)-cyclohexylgruppierung − besitzt, umsetzt, den nach Hydrolyse erhaltenen Alkohol der Formel (VII)

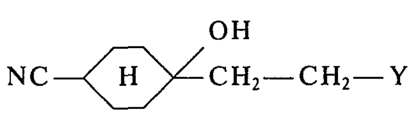

(VII)

dehydratisiert und in dem dabei entstehenden Gemisch aus Cyclohexen- bzw. Ethylidencyclohexanverbindungen die C=C-Doppelbindung selektiv hydriert, beispielsweise mit Diimin. Die Herstellung der Verbindungen der Formeln (Idd) und (Iii) erfolgt analog, wobei jedoch anstelle der Grignard-Verbindung (VI) eine Verbindung (VIa)

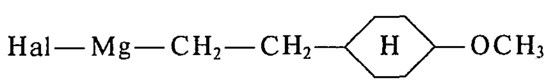

(VIa)

eingesetzt wird; dabei wird nach der Diimin-Reduktion die Methylethergruppe abgespalten, und die entstandene Hydroxylgruppe mit 4-R-Cyclohexancarbonsäure oder einem reaktiven Derivat davon verbessert.

Die in den beschriebenen Synthesen eingesetzten Ausgangsverbindungen der Formeln (II) bis (VIa) sind zum Teil bekannt, zum Teil können sie in an sich üblicher Weise analog zu den bekannten Ausgangsverbindungen nach Standardverfahren der präparativen organischen Chemie hergestellt werden.

Die erfindungsgemäßen flüssigkristallinen Dielektrika bestehen aus zwei oder mehr Komponenten, darunter mindestens eine der Formel (I); es können jedoch auch erfindungsgemäße Dielektrika ausschließlich aus Verbindungen der Formel (I) – abgesehen von gegebenenfalls zusätzlich anwesenden Dotier- oder Zusatzstoffen, die nicht notwendig auch selbst flüssigkristallin sind – bestehen. Gegebenenfalls verwendete weitere Komponenten sind vorzugsweise nematische oder nematogene Substanzen aus den Klassen der Azobenzole, Azoxybenzole, Biphenyle, ggf. partiell hydrierten Terphenyle oder Quaterphenyle, Schiff'schen Basen, insbesondere Benzyliden-Derivate, Phenylbenzoate, Phenylpyrimidine, Phenylcyclohexane, gegebenenfalls halogenierten Stilbene, Diphenylacetylen-Derivate, Diphenylnitrone, Phenyl- oder Cyclohexylnaphthaline, die auch in Naphthalinteil partiell hydriert sein oder Stickstoffatome enthalten können, sowie substituierte Zimtsäuren. Die wichtigsten als derartige weitere Komponenten in Frage kommenden Verbindungen lassen sich durch die Formel (VIII) charakterisieren:

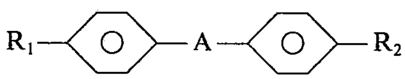 (VIII)

worin

A $—CH=CH—$ 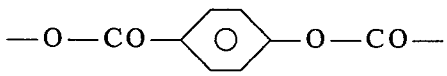

$—CM=CH—$

$—CH=CM—$

$—C≡C—$

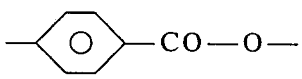 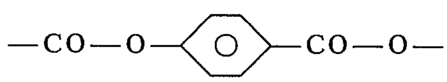

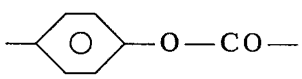

$—N=N—$ 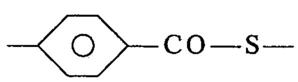

$—N(O)=N—$

$—N=N(O)—$ $—CH=N—$

$—CH_2—CH_2—$

$—O—CO—$ $—N=CH—$

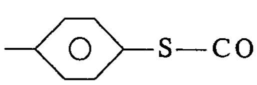 $—CH_2—O—$

$—O—CH_2—$

$—CO—O—$ $—CH=N(O)—$

$—S—CO—$ $—N(O)=CH—$

$—CO—S—$ 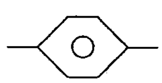 oder eine C–C-Einfachbindung

bedeutet. Weitere mögliche Komponenten der erfindungsgemäßen Dielektrika sind solche Verbindungen der Formel (VIII) in denen einer oder mehrere Phenylringe durch eine entsprechende Zahl von trans-Cyclohexylringen ersetzt sind; ferner kann auch einer dieser Ringe ein 2,5-disubstituierter Pyrimidinring, ein gegebenenfalls partiell hydriertes 2,6-disubstituiertes Naphthalin- oder Chinazolinsystem sein.

M bedeutet Halogen, vorzugsweise Cl, oder —CN. $R_1$ und $R_2$ sind gleich oder verschieden und können Alkyl-, Alkoxy-, Alkanoyl-, Alkanoyloxy- oder Alkoxycarbonyloxyreste mit bis zu 18, vorzugsweise bis zu 8 C-Atomen bedeuten; weiterhin kann auch einer dieser Reste trans-4-Alkylcyclohexyl, —CN, —NC, $-NO_2$, $-CF_3$ oder Halogen bedeuten.

Bei den meisten dieser Verbindungen sind $R_1$ und $R_2$ vorzugsweise verschieden, wobei einer der Reste meist eine Alkyl- oder Alkoxygruppe bedeutet. Aber auch eine große Zahl anderer Varianten der vorgesehenen Substituenten sind gebräuchlich. Viele solcher Substanzen sind im Handel erhältlich.

Die erfindungsgemäßen Dielektrika enthalten in der Regel mindestens 30, vorzugsweise 50—99, insbesondere 60—98 Gewichtsteile der Verbindungen der Formel (I) und gegebenenfalls (VIII). Hiervon entfallen bevorzugt mindestens 5 Gewichtsteile, meist auch 10 oder mehr Gewichtsteile auf eine oder mehrere Verbindungen der Formel (I). Es werden von der Erfindung auch solche flüssigkristallinen Dielektrika umfaßt, denen beispielsweise zu Dotierungszwecken nur weniger als 5 Gewichtsteile, zum Beispiel 0,1 bis 3 Gewichtsteile einer oder mehrerer Verbindungen der Formel (I) zugesetzt worden sind.

Die Herstellung der erfindungsgemäßen Dielektrika erfolgt in an sich üblicher Weise. In der Regel wird die gewünschte Menge der in geringerer Menge verwendeten Komponenten in der den Hauptbestandteil ausmachenden Komponente gelöst, zweckmäßig bei erhöhter Temperatur. Wenn dabei eine Temperatur oberhalb des Klärpunkts des Hauptbestandteils gewählt wird, kann die Vollständigkeit des Lösevorgangs besonders leicht beobachtet werden.

Es ist jedoch auch möglich, Lösungen des Komponenten der Formel (I) und gegebenenfalls (VIII) in einem geeigneten organischen Lösungsmittel, zum Beispiel Aceton, Chloroform oder Methanol, zu mischen und das Lösungsmittel nach gründlicher Durchmischung wieder zu entfernen, beispielsweise durch Destillation unter vermindertem Druck. Selbstverständlich muß bei dieser Verfahrensweise darauf geachtet werden, daß durch das Lösungsmittel keine Verunreinigungen oder unerwünschten Dotierungsstoffe eingeschleppt werden.

Durch geeignete Zusätze können die flüssigkristallinen Dielektrika nach der Erfindung so modifiziert werden, daß sie in allen bisher bekannt gewordenen Arten von Flüssigkristallanzeigeelementen verwendet werden können. Derartige Zusätze sind dem Fachmann bekannt und sind in der einschlägigen Literatur ausführlich beschrieben. Beispielsweise können Substanzen zur Veränderung der dielektrischen Anisotropie, der optischen Anisotropie, der Viskosität, der Leitfähigkeit und/oder der Orientierung der nematischen Phasen zugesetzt werden. Derartige Substanzen sind zum Beispiel in den DE-OS 2 209 127, 2 240 864, 2 321 632, 2 338 281, 2 535 046, 2 637 430, 2 702 598, 2 900 312 und 3 000 375 beschrieben.

Die folgenden Beispiele sollen die Erfindung erläutern. In den Beispielen bedeuten F. den Schmelzpunkt und K. den Klärpunkt einer flüssigkristallinen Substanz in Grad Celsius; Siedetemperaturen sind mit Kp. bezeichnet. Wenn nichts anderes angegeben ist, bedeuten Angaben von Teilen oder Prozent Gewichtsteile bzw. Gewichtsprozent.

## Beispiel 1

Zu einer Lösung von 1,25 g trans-4-Cyanocyclohexanol und 1,5 g 4-Dimethylaminopyridin in 50 ml Dichlormethan wird unter Rühren bei Raumtemperatur eine Lösung von 2,1 g 4-n-Pentylbenzoylchlorid in 20 ml Dichlormethan getropft. Das Reaktionsgemisch wird 2 Stunden bei Raumtemperatur gerührt, das Lösungsmittel unter vermindertem Druck abdestilliert, der Rückstand in 100 ml Diethylether aufgenommen, die erhaltene Lösung nacheinander mit je 20 ml 2 N Salzsäure und 5% wäßriger Natriumhydrogenkarbonatlösung ausgeschüttelt und über Magnesiumsulfat getrocknet. Der nach dem Eindampfen zurückbleibende 4-n-Pentylbenzoesäure(trans-4-cyanocyclohexyl)-ester wird aus Ethanol umkristallisiert; F. 28°, K. —60 (monotrop).

Analog werden hergestellt:

4-n-Heptylbenzoesäure-(trans-4-cyanocyclohexyl)-ester, F. 53°, K. —8°;
4-n-Nonylbenzoesäure-(trans-4-cyanocyclohexyl)-ester, F. 39°, K. —5°;
4-Ethoxybenzoesäure-(trans-4-cyanocyclohexyl)-ester,
4-n-Octyloxybenzoesäure-(trans-4-cyanocyclohexyl)-ester,
4-n-Propylbiphenyl-4'-carbonsäure-(trans-4-cyanocyclohexyl)-ester,
4-n-Hexylbiphenyl-4'-carbonsäure-(trans-4-cyanocyclohexyl)-ester,
4-(2-Methylbutyl)-biphenyl-4'-carbonsäure-(trans-4-cyanocyclohexyl)-ester,
4-n-Butyloxybiphenyl-4'-carbonsäure-(trans-4-cyanocyclohexyl)-ester,
4-n-Pentyloxybiphenyl-4'-carbonsäure-(trans-4-cyanocyclohexyl)-ester,
all-trans-4-(4-n-Propylcyclohexyl)-cyclohexancarbonsäure-(4-cyanocyclohexyl)-ester,
all-trans-4-(4-n-Heptylcyclohexyl)-cyclohexancarbonsäure-(4-cyanocyclohexyl)-ester,
4-(trans-4-n-Pentylcyclohexyl)-benzoesäure-(trans-4-cyanocyclohexyl)-ester, F. 104°, K. 138°;
4-(trans-4-n-Propylcyclohexyl)-benzoesäure-(trans-4-cyanocyclohexyl)-ester, F. 104°, K. 145°;
4-(trans-4-n-Heptylcyclohexyl)-benzoesäure-(trans-4-cyanocyclohexyl)-ester, F. 99°, K. 133°;

4-(trans-4-Methoxycyclohexyl)-benzoesäure-(trans-4-cyanocyclohexyl)-ester,
trans-4-Cyanocyclohexancarbonsäure-(4-ethylphenyl)-ester,
trans-4-Cyanocyclohexancarbonsäure-(4-n-pentylphenyl)-ester,
trans-4-Cyanocyclohexancarbonsäure-(4-n-decylphenyl)-ester,
trans-4-Cyanocyclohexancarbonsäure-[4-(2-ethylhexyloxyphenyl)]-ester,
trans-4-Cyanocyclohexancarbonsäure-[4-(trans-4-methylcyclohexyl)-phenyl]-ester,
trans-4-Cyanocyclohexancarbonsäure-[4-(trans-4-n-butylcyclohexyl)-phenyl]-ester,
trans-4-Cyanocyclohexancarbonsäure-[4-(trans-4-n-hexylcyclohexyl)-phenyl]-ester,
trans-4-Cyanocyclohexancarbonsäure-(4-n-pentylbiphenyl-4'-yl)-ester,
trans-4-Cyanocyclohexancarbonsäure-(4-ethylbiphenyl-4'-yl)-ester,
trans-4-Cyanocyclohexancarbonsäure-(4-n-propyloxybiphenyl-4'-yl)-ester,
trans-4-Cyanocyclohexancarbonsäure-(4-n-octyloxybiphenyl-4'-yl)-ester,
all-trans-4-Cyanocyclohexancarbonsäure-[4-(4-n-pentylcyclohexyl)-cyclohexyl]-ester,
all-trans-4-Cyanocyclohexancarbonsäure-[4-(4-n-hexylcyclohexyl)-cyclohexyl]-ester.

## Beispiel 2

Zu einer Lösung von 1,8 g trans-4-Bromcyclohexanol und 0,8 g Pyridin in 30 ml Diethylether wird bei Raumtemperatur eine Lösung von 2,1 g 4-n-Pentylbenzoylchlorid in 30 ml Diethylether getropft. Das Reaktionsgemisch wird 12 Stunden bei Raumtemperatur gerührt, vom ausgefallenen Pyridinhydrochlorid abfiltriert, das Filtrat nacheinander mit je 20 ml 2 N wäßriger Salzsäure und 5% wäßriger Natriumhydrogencarbonatlösung ausgeschüttelt und über Magnesiumsulfat getrocknet. Die so erhaltene etherische Lösung von 4-n-Pentylbenzoesäure-(trans-4-bromcyclohexyl)-ester wird bei 0° zu einer Lösung von 1,4 g Bortrifluorid-etherat in 50 ml Diethylether gegeben und das erhaltene Gemisch unter Rühren bei dieser Temperatur mit einer Lösung von 1,2 g Natriumborhydrid in 20 ml Diethylenglykoldimethylether versetzt. Das Reaktionsgemisch wird noch 1 Stunde bei 0° gerührt und dann eine weitere Stunde zum Sieden erhitzt. Nach dem Abkühlen wird das Reaktionsgemisch mit 100 ml Wasser ausgeschüttelt, die Etherphase abgetrennt und über Magnesiumsulfat getrocknet. Nach Abdestillieren des Lösungsmittels wird der zurückbleibende trans-4-Bromcyclohexyl-(4-n-Pentylbenzyl)-ether in 35 ml Ethanol aufgenommen und mit einer Lösung von 2 g Kaliumcyanid in 10 ml Wasser versetzt. Das Reaktionsgemisch wird 3 Stunden zum Sieden erhitzt, dann in 50 ml Wasser gegossen und das resultierende Gemisch zweimal mit je 60 ml Diethylether ausgeschüttelt. Die Etherextrakte werden über Magnesiumsulfat getrocknet, eingedampft und der zurückbleibende trans-4-Cyanocyclohexyl-(4-n-pentylbenzyl)-ether aus Petrolether (Siedebereich 30—40°C) umkristallisiert.

Analog werden hergestellt:

trans-4-Cyanocyclohexyl-(4-n-heptylbenzyl)-ether,
trans-4-Cyanocyclohexyl-(4-ethylbiphenylmethyl)-ether,
trans-4-Cyanocyclohexyl-[4-(trans-4-n-propylcyclohexyl)-benzyl]-ether,
all-trans-4-Cyanocyclohexyl-(4-n-butylcyclohexylmethyl)-ether,
all-trans-4-Cyanocyclohexyl-[4-(4-n-hexylcyclohexyl)-cyclohexylmethyl]-ether,
trans-4-Cyanocyclohexylmethyl-(4-n-octylphenyl)-ether,
all-trans-4-Cyanocyclohexylmethyl-(4-n-pentylcyclohexyl)-ether,
all-trans-4-Cyanocyclohexylmethyl-[4-(4-n-heptylcyclohexyl)-cyclohexyl]-ether,
trans-4-Cyanocyclohexylmethyl-(4-n-octyloxybiphenyl-4'-yl)-ether,
trans-4-Cyanocyclohexylmethyl-[4-(trans-4-n-pentylcyclohexyl)-phenyl]-ether,

## Beispiel 3

Zu einer aus 13 g 1-(4-n-Pentylphenyl)-2-bromethan und 1,3 g Magnesiumspänen in 200 ml Diethylether hergestellten Grignard-Lösung wird unter Rühren bei Raumtemperatur im Lauf von 1,5 Stunden eine Lösung von 6 g 4-Cyanocyclohexanon in 50 ml Diethylether getropft. Anschließend wird das Reaktionsgemisch 2 Stunden unter Rückfluß zum Sieden erhitzt und nach dem Abkühlen unter Rühren mit 150 ml 10% wäßriger Ammoniumchloridlösung versetzt. Die Etherphase wird abgetrennt, über Natriumsulfat getrocknet und eingedampft. Das zurückbleibende 1-(4-n-Pentylphenyl)-2-[4-Cyano-1-hydroxycyclohexyl-(1)]-ethan wird in 40 ml Aceton gelöst und diese Lösung mit 0,5 g p-Toluolsulfonsäure 1 Stunde zum Sieden erhitzt. Anschließend wird das Lösungsmittel abdestilliert und der Rückstand in 100 ml Ethanol aufgenommen. Nach Zusatz von 20 mg Kupfer (II) sulfat werden bei 0° nacheinander 23 g Hydrazinhydrat und 40 ml 30% wäßrige Wasserstoffperoxidlösung zugesetzt. Das Reaktionsgemisch wird 1 Stunde bei Raumtemperatur gerührt, in 500 ml Wasser gegossen und das so erhaltene 1-(4-n-Pentylphenyl)-2-(trans-4-cyanocyclohexyl)-ethan mit Diethylether extrahiert; die Extrakte werden mit Natriumsulfat getrocknet, eingedampft und der Rückstand aus Isopropanol umkristallisiert.

Analog werden hergestellt:

1-(4-n-Hexyloxyphenyl)-2-(trans-4-cyanocyclohexyl)-ethan,
1-(trans-4-n-Butylcyclohexyl)-2-(trans-4-cyanocyclohexyl)-ethan,
1-[4-(trans-4-n-Propylcyclohexyl)-phenyl]-2-(trans-4-cyanocyclohexyl)-ethan,
1-(4-n-Pentyloxybiphenyl-4'-yl)-2-(trans-4-cyanocyclohexyl)-ethan,
1-(4-n-Heptylbiphenyl-4'-yl)-2-(trans-4-cyanocyclohexyl)-ethan,
all-trans-1-[4-Ethylcyclohexyl)-cyclohexyl]-2-(4-cyanocyclohexyl)-ethan.

## Patentansprüche

1. Cyclohexylcarbonitrilderivate der Formel (I)

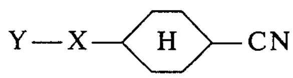

(I)

worin

Y 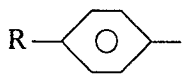 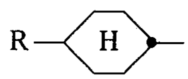 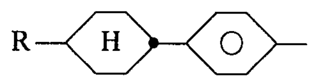

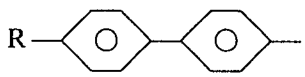 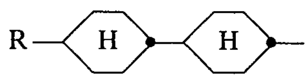

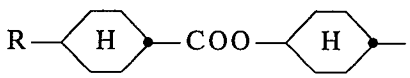 oder 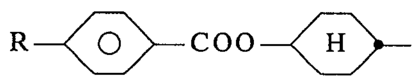

X $-COO-$, $-O-CO-$, $-CH_2O-$, $-OCH_2-$ oder $-CH_2-CH_2-$, und
R Alkyl oder Alkoxy mit 1–12 Kohlenstoffatomen

bedeutet, wobei jedoch Y nicht

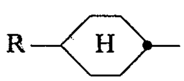 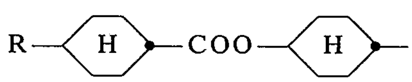

oder

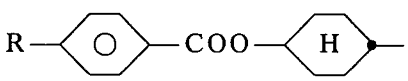

bedeutet, wenn X $-COO-$ oder $-O-CO-$ ist.

2. Verwendung der Cyclohexylcarbonitrilderivate der Formel (I) nach Anspruch 1 als Komponenten flüssigkristalliner Dielektrika.

3. Flüssigkristallines Dielektrikum mit mindestens 2 flüssigkristallinen Komponenten, dadurch gekennzeichnet, daß es mindestens ein Cyclohexylcarbonitrilderivat der Formel (I) nach Anspruch 1 enthält.

4. Elektrooptisches Anzeigeelement auf der Basis einer Flüssigkristallzelle, dadurch gekennzeichnet, daß es ein flüssigkristallines Dielektrikum nach Anspruch 3 enthält.

## Claims

1. Cyclohexylcarbonitrile derivatives of the formula (I)

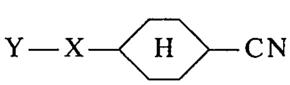

(I)

wherein

Y is 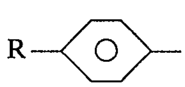 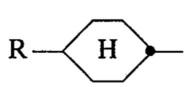 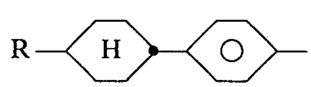

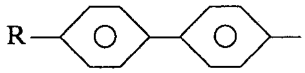 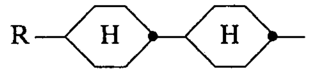

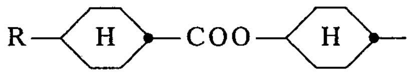 or 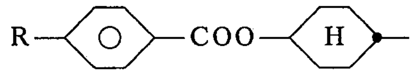

X is —COO—, —O—CO—, $-CH_2O-$, $-OCH_2-$ or $-CH_2CH_2-$, and
R is alkyl or alkoxy having 1—12 carbon atoms,

Y however, not being

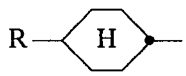 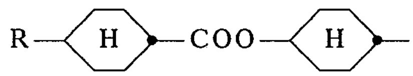

or

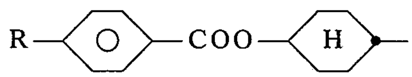

if X is —COO— or —O—CO—.

2. Use of cyclohexylcarbonitrile derivatives of the formula (I) according to Claim 1 as components of liquid-crystalline dielectrics.

3. Liquid-crystalline dielectric with at least 2 liquid-crystalline components, characterised in that it contains at least one cyclohexylcarbonitrile derivative of the formula (I) according to Claim 1.

4. Electro-optical display element based on a liquid crystal cell, characterised in that it contains a liquid-crystalline dielectric according to Claim 3.

## Revendications

1. Dérivés de cyclohexylcarbonitrile de formule (I)

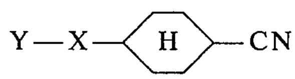 (I)

où Y représente

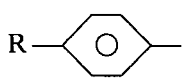 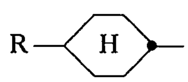 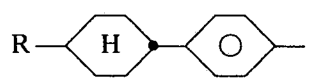

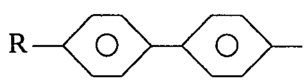 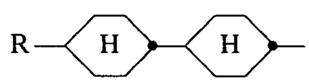

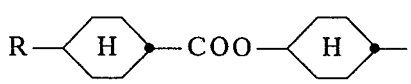 ou 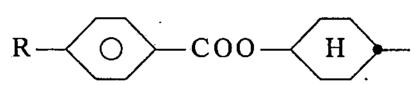

X représente

—COO—, —O—CO—, $-CH_2O-$, $-OCH_2-$ ou $-CH_2CH_2-$, et

R représente

un alcoyle ou un alcoxy en $C_1$ à $C_{12}$,

où cependant Y ne représente pas

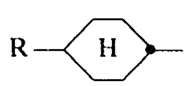 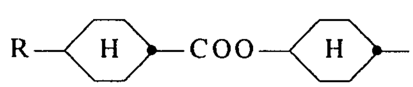

ou

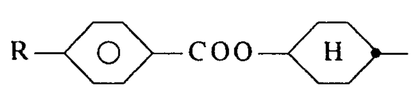

lorsque X représente —COO— ou —O—CO—.

2. Application des dérivés de cyclohexylcarbonitrile de formule (I) selon la revendication 1 comme composants de diélectriques à cristaux liquides.

3. Diélectrique à cristaux liquides ayant au moins 2 composants à cristaux liquides, caractérisé en ce qu'il contient au moins un dérivé de cyclohexylcarbonitrile de formule (I) selon la revendication 1.

4. Elément d'affichage électro-optique à base d'une cellule à cristaux liquides, caractérisé en ce qu'il contient un diélectrique à cristaux liquides selon la revendication 3.